# EUROPEAN PATENT SPECIFICATION

(11) **EP 1 929 968 B1**
(45) Date of publication and mention of the grant of the patent: **29.05.2013**
(21) Application number: 07020063.9
(22) Date of filing: 12.10.2007
(51) Int. Cl.: A61B 18/14, B05B 1/34, A61B 18/00

(54) **Electrosurgical instrument**
Elektrochirurgisches Instrument
Instrument électro-chirurgical

(30) Priority: 04.12.2006 US 633346
(43) Date of publication of application: 11.06.2008
(73) Proprietor: OLYMPUS MEDICAL SYSTEMS CORP., Tokyo 151-0072 (JP)
(72) Inventor: Iida, Koji, Tokyo 151-0072 (JP); Wakamatsu, Mai, Tokyo 151-0072 (JP); Takashino, Tomoyuki, Tokyo 151-0072 (JP)
(74) Representative: von Hellfeld, Axel

(56) References cited:
- EP-A- 0 740 926
- WO-A-97/24969
- WO-A-98/14131
- GB-A- 191 413 757
- US-A- 5 167 659
- US-A- 5 725 524
- US-A1- 2002 082 666
- US-B1- 6 354 519

## Description

### BACKGROUND OF THE INVENTION

### 1. Field of the Invention

The present invention relates to an electrosurgical instrument using both high-frequency electric energy and a conductive fluid for coagulating a surface layer of a living tissue by electric discharge.

### 2. Description of the Related Art

Recently, as an electrosurgical instrument using both high-frequency electric energy and a conductive fluid for coagulating a surface layer of a living tissue by electric discharge, a treatment instrument for an abdominal surgery, a treatment instrument to be used with a rigid endoscope and a flexible treatment instrument to be used with a flexible endoscope are known.

In Japanese Patent No. 3318733, for example, a surgical device is proposed for incision or coagulation of a living tissue by conducting a high-frequency electric current between a nozzle electrode and a portion to be treated through a conductive fluid jet injected from the nozzle electrode toward the portion to be treated of the living tissue.

Specifically, in the surgical device disclosed in Japanese patent No. 3318733, such a construction is provided that, after a discharge is formed between the nozzle electrode and the portion to be treated, the fluid jet is injected from the nozzle electrode toward the portion to be treated of a living tissue so as to pass the through discharge column for incision/coagulation of the living tissue in a non-contact manner from the nozzle electrode using discharge current energy flowing to the portion to be treated through the fluid jet.

Moreover, Japanese Examined Patent Application Publication No. 7-034805 discloses a coagulating device for non-contact hemostatic coagulation of a living tissue from an active electrode by conducting a high-frequency current from the active electrode to the living tissue through the conductive fluid while atomizing the conductive fluid mixed with gas from a distal hole of the active electrode.

However, in Japanese Patent No. 3318733, since electric discharge is performed from the nozzle electrode, the nozzle electrode can be molten/worn by the discharge. If the nozzle electrode is worn, the atomizing shape of the conductive fluid jet injected from the nozzle electrode is changed from that at the normal time, which causes a problem that a coagulated state of a living tissue is deteriorated.

Also, Japanese Patent No. 3318733 has a construction in which a water flow of the conductive fluid jet is focused by an insulating covering member, but it has a problem that the discharge state becomes unstable if water drops adheres to the distal end of the insulating covering member.

Moreover, with Japanese Patent No. 3318733, since the water flow of the conductive fluid jet is used in the focused state, the fluid jet can be injected only in a narrow range of the living tissue. In other words, since the atomizing range can not be widened structurally, it has a problem that the coagulation range in the living tissue is narrow all the time.

In Japanese Examined Patent Application Publication No. 7-034805, too, if the active electrode is worn, the atomizing shape of the conductive liquid injected from the active electrode is changed from that at the normal time, which causes a problem that the coagulated state of the living tissue is deteriorated and also, it has a problem that water drops adhering around the nozzle covering the active electrode makes the discharge state unstable.

EP 0 740 926 A2 discloses an electrosurgical apparatus for generating an electric arch. Such apparatus is used for contactless surface coagulation of bio tissue by a high frequency cutting electrode. For generating an ionized path in order to support generation of an electric arch an aerosol jet is generated in a tubular body exiting at the distal end of the body. The high frequency surgical cutting electrode is disposed coaxially to the tubular body at the distal end, but is slightly spaced apart from the distal end of the tubular body towards the inner of the tubular body. At the proximal end of the body, supply lines for supplying fluid to the inner of the body are disposed. Such supply lines located at the proximal end comprise openings from which a sodium chloride solution is sprayed into the inner of the body.

The present invention was made in view of the above problems and its object is to provide an electrosurgical instrument performing discharge using high-frequency electric energy while atomizing a conductive fluid by a nozzle or the like so as to coagulate a living tissue, which can prevent ablation or damage of the nozzle by the discharge and can obtain a favorable coagulation state of a living tissue over a wide range by realizing a stable discharge state through prevention of adhesion of water drops around the nozzle.

### SUMMARY OF THE INVENTION

In brief, an electrosurgical instrument of the present invention is as per claim 1.

The above and other objects, features and advantages of the invention will become more clearly understood from the following description referring to the accompanying drawings.

### BRIEF DESCRIPTION OF THE DRAWINGS

Fig. 1 is a block diagram showing a treatment instrument system provided with a treatment instrument showing a first embodiment;
Fig. 2 is an enlarged perspective view of a treatment instrument in Fig. 1;
Fig. 3 is a partial sectional view of the distal side of the treatment instrument in Fig. 2;
Fig. 4 is a sectional view of a swirl member in Fig. 3 seen from the IV direction;
Fig. 5 is a partial sectional view of the proximal side of the treatment instrument in Fig. 2;
Fig. 6 is a partial sectional view of the distal side of a treatment instrument showing a second embodiment;
Fig. 7 is a partial sectional view of the distal side of a treatment instrument showing a third embodiment;
Fig. 8 is a sectional view of a swirl member along the VIII-VIII line in Fig. 7;
Fig. 9 is a partial perspective view of the distal side of a treatment instrument showing a fourth embodiment;
Fig. 10 is a partial sectional view showing a construction of the distal side of a treatment instrument showing a fifth embodiment;
Fig. 11 is a partial sectional view showing a construction of the proximal side of a treatment instrument showing the fifth embodiment;
Fig. 12 is a partial sectional view showing a construction of the distal side of a treatment instrument showing a sixth embodiment;
Fig. 13 is a partial sectional view showing a construction of the distal side of a treatment instrument showing a seventh embodiment;
Fig. 14 is a partial sectional view showing a construction of the distal side of a treatment instrument showing an eighth embodiment;
Fig. 15 is a perspective view showing a construction of a treatment instrument showing a ninth embodiment;
Fig. 16 is a partial sectional view of the distal portion side of a treatment instrument in Fig. 15;
Fig. 17 is a partial sectional view showing a construction of the distal side of a treatment instrument showing a tenth embodiment;
Fig. 18 is a block diagram showing a treatment instrument system provided with a treatment instrument showing an eleventh embodiment;
Fig. 19 is a partial sectional view showing a construction of the distal side of the treatment instrument in Fig. 18;
Fig. 20 is a partial sectional view showing a variation of the shape of a mist generation portion of the treatment instrument in Fig. 19;
Fig. 21 is a partial sectional view showing another variation of the shape of a mist generation portion of the treatment instrument in Fig. 19;
Fig. 22 is a partial sectional view showing still another variation of the shape of a mist generation portion of the treatment instrument in Fig. 19;
Fig. 23 is a partial sectional view showing a construction of the distal side of a treatment instrument showing a twelfth embodiment;
Fig. 24 is a block diagram showing a treatment instrument system provided with a treatment instrument of a thirteenth embodiment;
Fig. 25 is a partial sectional view showing a construction of the distal side of the treatment instrument in Fig. 24;
Fig. 26 is a partial sectional view showing a construction of the distal side of a treatment instrument showing a fourteenth embodiment;
Fig. 27 is a partial sectional view showing a construction of the proximal side of a treatment instrument showing a fourteenth embodiment;
Fig. 28 is a partial sectional view showing a construction of the distal side of a treatment instrument showing a fifteenth embodiment;
Fig. 29 is a partial sectional view showing a construction of the proximal side of a treatment instrument showing a fifteenth embodiment;
Fig. 30 is a partial sectional view showing a treatment instrument showing a sixteenth embodiment together with a liquid feed pump;
Fig. 31 is a partial sectional view showing a treatment instrument showing a seventeenth embodiment together with a liquid feed pump;
Fig. 32 is a perspective view showing a handpiece for an abdominal surgery;
Fig. 33 is a perspective view showing a treatment instrument for a surgery under laparoscope; and
Fig. 34 is a partial sectional view showing a variation of a vibration probe in Fig. 25.

### DETAILED DESCRIPTION OF THE PREFERRED EMBODIMENTS

Embodiments of electrosurgical instruments, some of which are embodiments of the present invention will as per the claims, be described below referring to the attached drawings. It is to be noted that in the following embodiments, as an electrosurgical instrument for coagulating a surface layer of a living tissue by discharge using both high-frequency electric energy and a conductive fluid, description will be made using a treatment instrument for medical care as an example. Also, in the description below for the treatment instrument, the side to be inserted into a body cavity is referred to as the distal side and the operation portion side as the proximal side.

### (First embodiment)

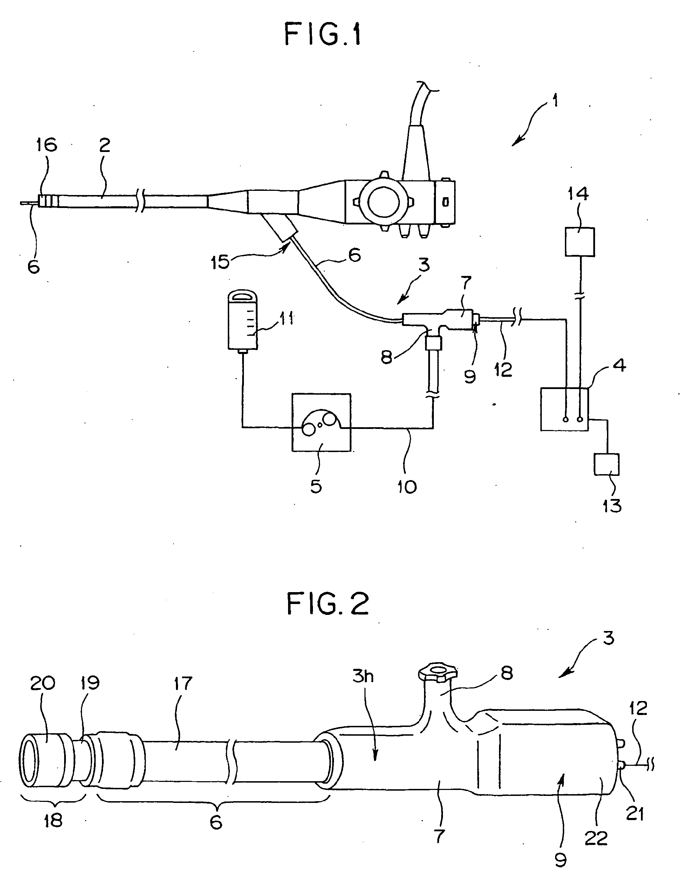

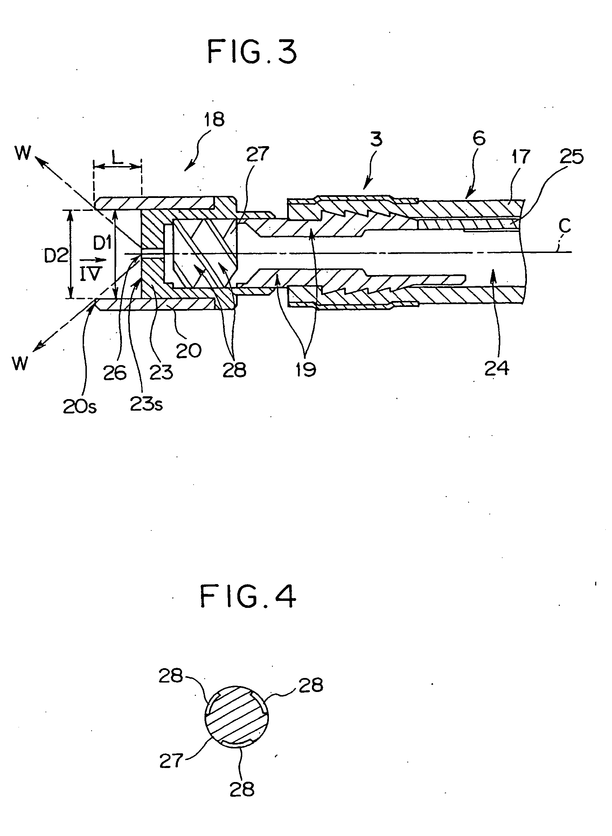

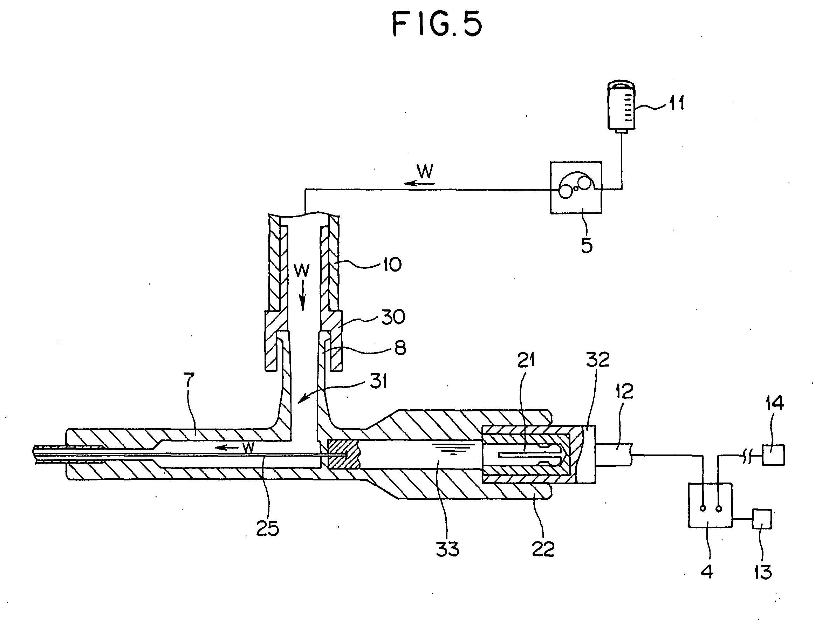

Fig. 1 is a block diagram showing a treatment instrument system provided with a treatment instrument showing this embodiment, Fig. 2 is an enlarged perspective view of the treatment instrument in Fig. 1, Fig. 3 is a partial sectional view of the distal side of the treatment instrument in Fig. 2, Fig. 4 is a sectional view of a swirl member in Fig. 3 seen from the IV direction, and Fig. 5 is a partial sectional view of the proximal side of the treatment instrument in Fig. 2.

As shown in Fig. 1, a treatment instrument system 1 mainly comprises a treatment instrument 3 capable of insertion/withdrawal with respect to a treatment channel 15 of an endoscope 2, a high-frequency power source 4, which is a power source connectable to the treatment instrument 3 and a liquid feed pump 5.

In the treatment instrument 3, a treatment instrument body 3h (See Fig. 2) is formed of an elongated tubular member, and the instrument body 3h mainly comprises an elongated insertion portion 6 and a proximal portion 7, which is connected to the proximal side of the insertion portion 6.

At the proximal portion 7, a connector 8 for liquid feed tube, which is a liquid feed connector, and a cable connection portion 9 are provided.

One end of a liquid feed tube 10 having the liquid feed pump 5, which is a supply pump, interposed at the middle position is connected to the connector 8 for liquid feed tube. Specifically, as shown in Fig. 5, a tube base 30 is provided at one end of the liquid feed tube 10, and this tube base 30 is connected to the connector 8 for liquid feed tube. By this, the inside of the liquid feed tube 10 communicates with a liquid feed passage 31 inside the connector 8 for liquid feed tube and a flow passage 24 inside a tube 17, which will be described later.

Also, a liquid feed container 11, which is a liquid supply source in which a liquid for liquid feed, which is a conductive fluid (hereinafter abbreviated simply as a liquid) W, is reserved is connected to the other end of the liquid feed tube 10. The liquid W is preferably an electrolytic solution such as normal saline solution, for example.

The connector 8 for liquid feed tube is to flow the liquid fed by the liquid feed pump 5 from the liquid feed container 11 through the liquid feed tube 10 into the liquid feed passage 31 inside the treatment instrument 3 (See Fig. 5) and the flow passage 24 (See Fig. 3).

To the cable connection portion 9, the other end of a conductive cable 12 having one end connected to the high-frequency power source 4 is connected. To the high-frequency power source 4, a foot switch 13 for controlling output of the treatment instrument and a return electrode 14 stuck to the body surface of a subject are connected.

Also, at the cable connection portion 9, as shown in Fig. 2, a plug 21 to which the other end of the conductive cable 12 is connected and a plug cover 22 partially covering the periphery of the plug 21 are provided.

As shown in Fig. 5, a cable connector 32 is provided at the other end of the conductive cable 12, and by the cable connector 32, the other end of the conductive cable 12 and the plug 21 are connected to each other.

The insertion portion 6 is formed with a diameter capable of insertion/withdrawal with respect to the treatment channel 15 of the endoscope 2 and is formed with a length sufficient to be projected from a distal portion 16 of the endoscope 2, when it is inserted into the treatment channel 15, 1 to 3 meters, for example.

Also, as shown in Fig. 2, the insertion portion 6 is comprised by a hollow flexible tube 17, and a treatment portion 18 is connected at the distal end of the insertion portion 6 through a tube connection portion 19. Also, at the treatment portion 18, an electrode 20 formed of a cylindrical conductive member is provided.

As shown in Fig. 3, a nozzle portion 23 is provided on the inside surface of a cylindrical electrode 20, having a nozzle hole 26 with a small diameter formed at the distal end and a hole with a diameter larger than that of the nozzle hole 26 formed at the rear end. In other words, the electrode 20 is fixed to the nozzle portion 23 so as to cover the outside of the nozzle portion 23 with the nozzle hole 26 of the nozzle portion 23 as a center axis C. That is, the center axis of the nozzle hole 26 and the center axis of the electrode 20 coincide with each other as the center axis C. This enables the electric discharge described later from the electrode 20 to be stabilized.

Moreover, the electrode 20 is fixed to the nozzle portion 23 so that a distal end 20s of the electrode 20 is located protruding toward the distal side from a distal face 23s of the nozzle portion 23 by a distance L.

Also, an inner diameter D1 of the distal end 20s of the electrode 20 is formed at a diameter equal to or larger than an outer diameter D2 (D1 ≧ D2) of the liquid W at the distal end 20s injected from the nozzle hole 26 of the nozzle portion 23.

To the inside of the rear end of the nozzle portion 23, the distal end of the tube connection portion 19 is connected, and to the outside of the tube connection portion 19, the distal end of the tube 17 is connected. Also, at the rear end of the tube connection portion 19, a distal end of a lead wire 25, which is an electric conductive member, extending into a flow passage 24 inside the tube 17 is electrically connected.

The rear end of the lead wire 25 is, as shown in Fig. 5, connected to a plug body 33 of the plug 21 through the flow passage 24, the liquid feed passage 31. By this, the lead wire 25 transmits the high-frequency current, which is high-frequency electric energy supplied from the high-frequency power source 4, from the proximal side to the distal side in the treatment instrument body 3h, specifically, from the plug 21 to the electrode 20.

At the distal side of the nozzle portion 23, the nozzle hole 26 for injecting the liquid W in the atomized state is provided. The nozzle hole 26 is formed in a straight hole with approximately φ0.1 to φ0.5 millimeters, considering liquid flow rate/liquid pressure in this embodiment.

Also, inside of the hole with a diameter larger than that of the nozzle hole 26 at the rear end side of the nozzle portion 23, a swirl member 27 in the column shape is provided. On the outside of the swirl member 27, two or three spiral flow passages 28 arranged in the axial symmetry are formed as shown in Figs. 3 and 4.

The swirl member 27 is to introduce a swirling flow to the nozzle hole 26 of the nozzle portion 23 by generating the swirling flow by the flow passage 28 in the liquid W flowing into the flow passage 24 by the liquid feed pump 5.

From the above construction, the nozzle portion 23 injects the swirling flow introduced from the swirl member 27 in the atomized state from the treatment portion 18 so that the outer diameter D2 of the liquid W at the distal end 20s of the electrode 20 becomes equal to or smaller than the inner diameter D 1 of the distal end 20s of the electrode 20 (D2 ≦ D1).

Moreover, from the above construction, the electrode 20 is to discharge the high-frequency current transmitted through the lead wire 25 along the atomized-state liquid W injected from the nozzle portion 23.

Next, operation of the so constructed treatment instrument 3 in this embodiment will be described.

First, when the liquid feed pump 5 is driven, the liquid W reserved in the liquid feed container 11 is fed to the treatment instrument 3. Specifically the liquid is fed in the order of the liquid feed container 11, the liquid feed tube 10, the liquid feed passage 31, the flow passage 24, the tube connection portion 19, the flow passage 28 of the swirl member 27 and the nozzle hole 26.

At this time, at the nozzle portion 23, since the swirling flow is generated in the liquid W by the spiral flow passage 28 of the swirl member 27, the liquid W is made into the atomized state when it is discharged from the nozzle hole 26 into the air, and the atomized-state liquid W is injected toward a tissue to be treated.

After injection of the liquid W from the nozzle hole 26, the foot switch 13 is operated. As a result, the high-frequency current is supplied from the high-frequency power source 4 to the treatment instrument 3. Specifically, the high-frequency current is supplied in the order of the high-frequency power source 4, the conductive cable 12, the plug 21, the plug body 33, the lead wire 25, the tube connection portion 19, the nozzle portion 23 and the electrode 20.

It is to be noted that it may be so constructed that both the liquid feed pump 5 and the high-frequency power source 4 are driven at the same time by the foot switch 13 by connection between the liquid feed pump 5 and the high-frequency power source 4 through a communication cable, not shown.

Then, the supplied high-frequency current is discharged by the electrode 20 along the atomized-state liquid W injected from the nozzle portion 23. Specifically, by the liquid W injected from the nozzle hole 26 of the nozzle portion 23, an atomization space is formed between the electrode 20 and the target tissue, and after the atomization space is made into a conductive passage with a low impedance, stable discharge is generated along the atomization from the entire circumference of the distal end 20s of the electrode 20 which is closest to the target tissue. As a result, a treatment such as coagulation is performed at the target tissue.

Here, at the distal end 20s of the electrode 20, the dimensions of the nozzle hole 26, the swirl member 27 and the inner diameter D1 of the electrode 20 are designed so that the inner diameter D1 of the electrode 20 is equal to or larger than the outer diameter D2 of the liquid W (D1 ≧ D2), and since the distal end 20s of the electrode 20 is located protruding from the distal face 23s of the nozzle portion 23 by the distance L, the liquid W injected from the nozzle hole 26 does not adhere to the inside surface or the distal end 20s of the electrode 20. That is, no such phenomenon that obstructs discharge would occur to make the discharge unstable.

Also, since at injection of the liquid W, the target tissue is cooled and coagulated by the injected liquid, when the liquid W is injected from the nozzle hole 26, if the liquid flow rate of the liquid W is large, coagulation performance becomes weak, while if the flow rate is small, coagulation performance becomes strong. Also, if the outer diameter D2 of the liquid W gets larger, a range of discharge is expanded and the coagulation range is widened, while if D2 gets smaller, the coagulation range is narrowed.

Finally, the high-frequency current after discharge is returned from the target tissue to the high-frequency power source 4 through a return electrode 14 adhered to the body surface of a patient.

In this way, in this embodiment, the distal end 20s of the electrode 20 is shown as being located protruding from the distal face 23s of the nozzle portion 23 toward the distal side by L.

According to this, since discharge is generated from the distal end 20s of the electrode 20 located at the closest to the target tissue, discharge from the nozzle portion 23 can be prevented, and ablation or damage of the nozzle portion 23 can be prevented. Thus, a favorable coagulation can be obtained over a wide range of the target tissue all the time without change over time of the atomizing shape of the liquid W.

Also, by setting the inner diameter D1 of the distal end 20s of the electrode 20 equal to or larger than the outer diameter D2 of the liquid W at the distal end 20s injected from the nozzle 26, the water drops of the liquid W does not adhere to the electrode 20, by which a stable discharge state for the target tissue can be realized and as a result, favorable coagulation can be obtained over a wide range of the target tissue all the time.

### (Second embodiment)

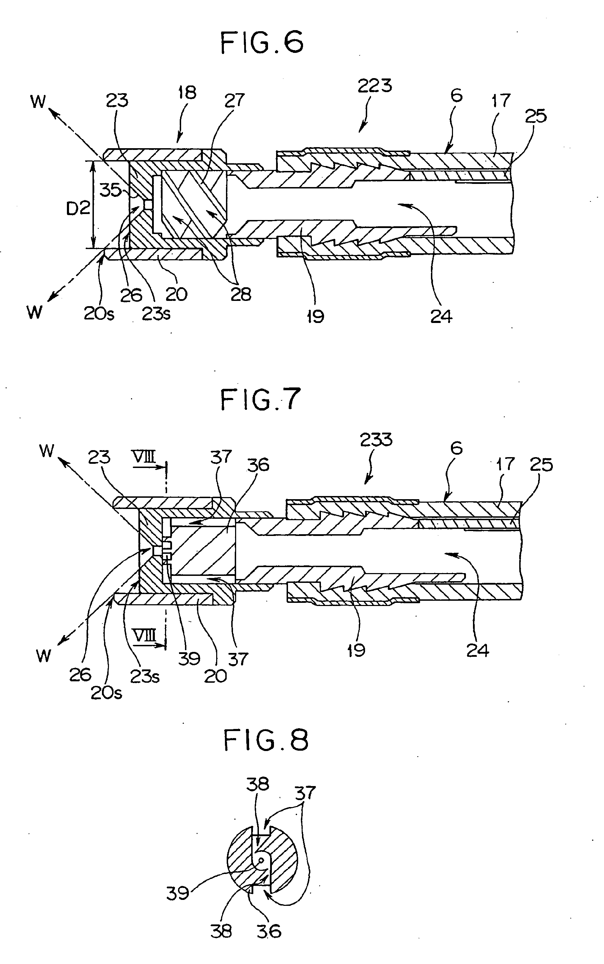

Fig. 6 is a partial sectional view of the distal side of a treatment instrument showing this embodiment.

In the construction of a treatment instrument 223 of this embodiment, the shape of the nozzle hole 26 is different from that of the treatment instrument 3 in the first embodiment shown in Figs. 1 to 5. Thus, only the difference will be described, and the same reference numerals are given to the same components as those in the first embodiment and the description will be omitted.

In the above-mentioned first embodiment, the nozzle hole 26 was shown to be formed into a straight hole with approximately φ0.1 to φ0.5 millimeters. Not being limited to this, in the treatment instrument 223 of this embodiment, the nozzle hole 26 is formed in the conical shape formed so that it gets wider from the proximal side to the distal side. In other words, a conical portion 35 is formed at the nozzle hole 26. It is to be noted that the other constructions are the same as those in the above-mentioned first embodiment.

If the conical portion 35 is formed at the nozzle hole 26 in this way, the outer diameter D2 of the liquid W at the distal end 20s of the electrode 20 can be made larger than that in the first embodiment by the conical portion 35. In this case, in line with expansion of the outer diameter D2, the inner diameter D1 of the electrode 20 and the distance L between the distal face 23s of the nozzle portion 23 and the distal end 20s of the electrode 20 should be adjusted with respect to the treatment instrument 3 in the first embodiment.

If the outer diameter D2 is larger, the discharge range is expanded and the coagulation range is widened. That is, the coagulation range for the living tissue can be made wider than that in the first embodiment. Also, by changing only the dimension of the conical portion 35, the atomizing range of the liquid W can be easily set according to the required coagulation range in the living tissue. The other effects are the same as those in the above-mentioned first embodiment.

### (Third embodiment)

Fig. 7 is a partial sectional view of the distal side of a treatment instrument showing this embodiment, and Fig. 8 is a sectional view of a swirl member along the VIII-VIII line in Fig. 7.

The construction of a treatment instrument 233 of this embodiment is different from the treatment instrument 223 in the second embodiment shown in Fig. 6 in the shape of the swirl member. Thus, only the difference will be described, and the same reference numerals are given to the same components as those in the second embodiment and the description will be omitted.

In this embodiment, as shown in Figs. 7 and 8, a swirl member 36 comprises two straight flow passages 37 in the longitudinal axis direction connecting the distal side to the proximal side formed outside of the swirl member 36, two flow passages 38 formed inside the swirl member 36 and communicating with the flow passages 37, and a swirling portion 39 provided inside the swirl member 36 and forming a swirling flow of the liquid W by mixing the flow of the liquid W in the flow passages 37 and the flow passages 38.

In the so constructed swirl member 36, the liquid W having advanced to the distal side in the flow passages 37 is made into the swirling flow in the swirling portion 39 through the flow passages 38. The liquid W made into the swirling flow is injected from the nozzle hole 26.

According to this, the swirl member can be formed in the relatively simplified structure than that in the above-mentioned first and the second embodiments. The other effects are the same as those in the above-mentioned second embodiment.

### (Fourth embodiment)

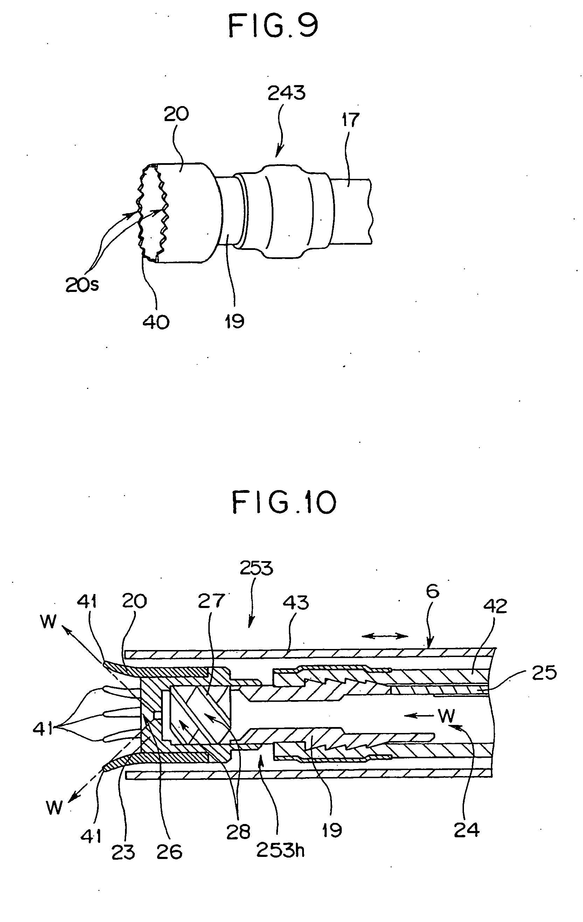

Fig. 9 is a partial perspective view of the distal side of a treatment instrument showing this embodiment.

The construction of a treatment instrument 243 of this embodiment is different from the treatment instrument 3 in the first embodiment shown in Figs. 1 to 5 in the shape of the electrode. Thus, only the difference will be described, and the same reference numerals are given to the same components as those in the first embodiment and the description will be omitted.

In this embodiment, as shown in Fig. 9, projections 40 with the triangular sectional shape, for example, are formed in the circumferential state at the distal end 20s of the electrode 20.

According to this, discharge is generated from each of the projections 40 at start of the discharge, and once the discharge is generated, the discharge is generated from the entire circumference of the distal end 20s of the electrode 20. Thus, discharge is more easily generated by the projections 40 than the electrode 20 in the above-mentioned first embodiment, and as a result, the distance between the target tissue and the electrode 20 can be made longer. The other effects are the same as those in the above-mentioned first embodiment.

### (Fifth embodiment)

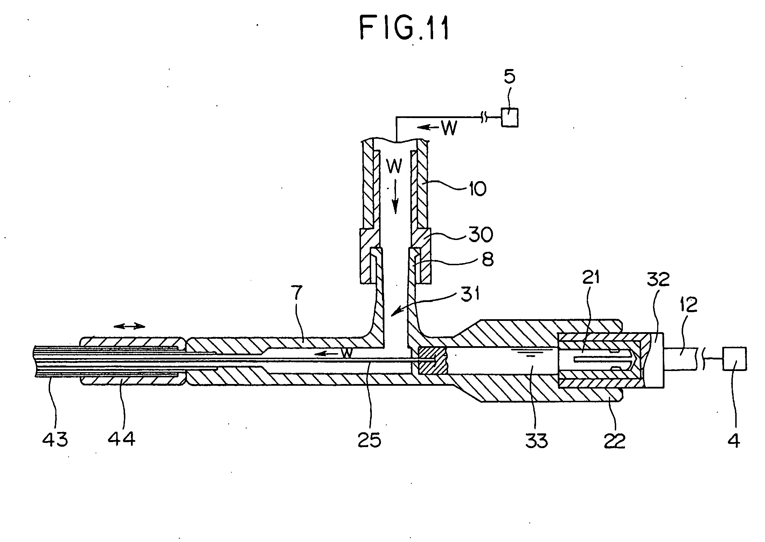

Fig. 10 is a partial sectional view showing the construction of the distal side of a treatment instrument showing this embodiment, and Fig. 11 is a partial sectional view showing the construction of the proximal side of the treatment instrument showing this embodiment.

The construction of a treatment instrument 253 of this embodiment is different from the treatment instrument 243 in the fourth embodiment shown in Fig. 6 in the point that a protective tube covers the outside of the treatment instrument body. Thus, only the difference will be described, and the same reference numerals are given to the same components as those in the fourth embodiment and the description will be omitted.

In this embodiment, as shown in Fig. 10, projections 41 formed at the distal end 20s of the electrode 20 are formed extending farther toward the distal side as compared with the above-mentioned fourth embodiment, and a protective tube 43 protecting the projections 41 covers the outside of a treatment instrument body 253h, which is a tubular member, capable of moving forward/backward so as to have a space between it and the treatment instrument body 253h. The tube 42 has the same construction and connection mode as those of the above-mentioned tube 17.

Also, as shown in Fig. 11, at the proximal side of the protective tube 43 and the distal side of the proximal portion 7, a protective tube operation portion 44 for operating the forward/backward movement of the protective tube 43 is provided.

Next, operation of this embodiment will be described. First, when the treatment instrument 253 is inserted into the treatment channel 15 of the endoscope 2, the protective tube 43 is slid and moved by the protective tube operation portion 44 toward the distal side till it covers the projections 41 in order to protect the projections 41 from damage.

When the treatment instrument 253 is inserted into the treatment channel 15 of the endoscope 2 for treatment, the protective tube 43 is slid and moved by the protective tube operation portion 44 toward the proximal side as shown in Fig. 10 so that the projections 41 are exposed in a body cavity.

According to such construction and operation, since the length of the projections 41 is longer than those in the fourth embodiment, discharge is easily generated and the distance between the target tissue and the electrode 20 can be made longer. The projections 41 might be worn by discharge, but since the length of the projections 41 is longer, durability is higher than the fourth embodiment. The other effects are the same as those of the above mentioned fourth embodiment.

### (Sixth embodiment)

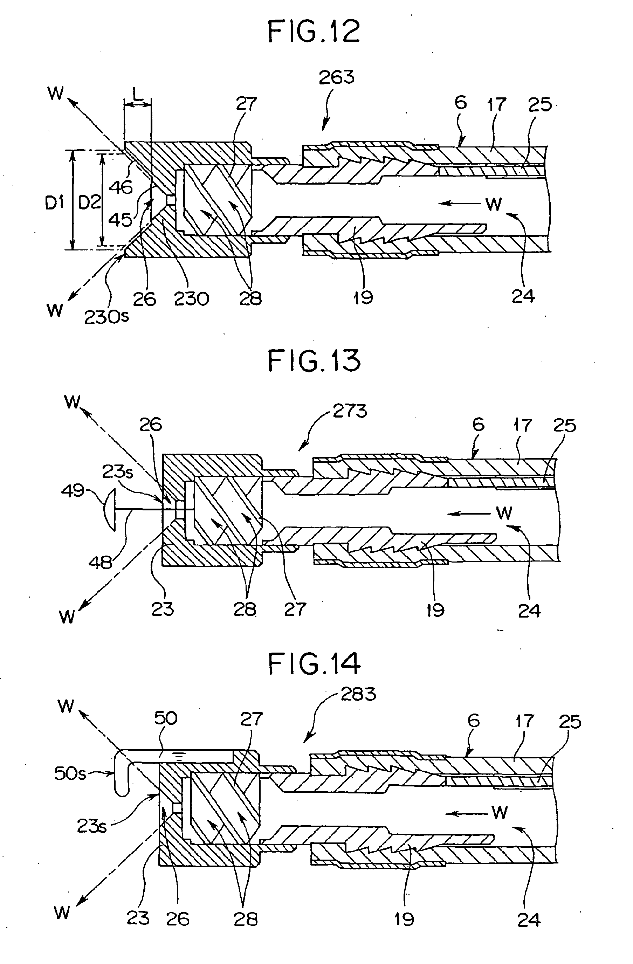

Fig. 12 is a partial sectional view showing the construction of the distal side of a treatment instrument showing this embodiment. The construction of a treatment instrument 263 of this embodiment is different from the treatment instrument 223 of the second embodiment shown in Fig. 6 in the point that the electrode and the nozzle portion are integrally formed. Thus, only the difference will be described, and the same reference numerals are given to the same components as those in the first embodiment and the description will be omitted.

As shown in Fig. 12, the nozzle portion 23 is constructed of a conductive member and formed integrally with the electrode 20 as an integral member 230 with the sectional shape having a conical recess portion formed at the distal end.

In more detail, at the distal end of the integral member 230, the conical nozzle hole 26 is formed in which a conical portion 45 formed as getting wider from the proximal side toward the distal side is formed. Moreover, at the distal side of the integral member 230 closer to the distal side than the nozzle hole 26, a conical hole is formed, in which a conical portion 46 with an opening diameter at the proximal end wider than that of the distal opening of the conical portion 45 is formed, as getting wider form the proximal side to the distal side.

By this conical portion 46, the inner diameter D1 of a distal end 230s of the integral member 230 corresponding to the distal end 20s of the electrode 20 of the first embodiment becomes equal to or larger than the outer diameter D2 of the liquid W injected from the nozzle hole 26 along the conical portions 45, 46 at the distal end 230s of the integral member 230 (D1 ≧ D2).

In this embodiment, too, the distal end 230s of the integral member 230 is located closer to the distal side than the distal end of the nozzle hole 26 at the integral member 230 corresponding to the distal face 23s of the nozzle portion 23 in the first embodiment.

According to the above construction, even if the nozzle portion 23 and the electrode 20 are formed integrally, the same effects as those of the above-mentioned second embodiment can be obtained. That is, since the water drop of the liquid W due to atomization does not adhere to the conical portion 46, stable discharge state can be realized for the target tissue, and as a result, favorable coagulation for the target tissue can be obtained over a wide range all the time.

### (Seventh embodiment)

Fig. 13 is a partial sectional view showing the construction of the distal side of a treatment instrument showing this embodiment. The construction of a treatment instrument 273 of this embodiment is different from the treatment instrument 223 of the second embodiment shown in Fig. 6 in the shape of the electrode. Thus, only the difference will be described, and the same reference numerals are given to the same components as those in the second embodiment and the description will be omitted.

In this embodiment, the electrode is not disposed at the treatment instrument 223 with covering the nozzle portion 23 by the cylindrical electrode 20 as shown in the above-mentioned second embodiment, but the electrode is disposed at a treatment instrument 273 by fixing the electrode to the distal end of a support rod extending toward the distal side from the nozzle hole 26.

Specifically, as shown in Fig. 13, a support rod 48, which is a conductive rod-shaped member, is provided in the treatment instrument 273 so that it protrudes from the distal face 23s of the nozzle portion 23 toward the distal side through the nozzle hole 26 from the swirl member 27, and an umbrella state electrode 49 is provided at the distal end of the support rod 48.

In this case, the swirl member 27 and the nozzle portion 23 are electrically connected since the swirl member 27 is pressed toward the distal side by the tube connection portion 19.

According to this construction, the high-frequency current transmitted by the lead wire 25 is transmitted in the order of the tube connection portion 19, the nozzle portion 23, the swirl member 27 and the support rod 48 to the electrode 49, from which discharge is carried out.

Thus, in this embodiment, since the electrode 49 is located protruding toward the distal side from the distal face 23s of the nozzle portion 23, the same effects as those of the above-mentioned second embodiment can be obtained.

Also, since the area of the electrode 49 is small, the discharge range is narrow, and the coagulation range can be limited. On the other hand, since discharge is easily generated from the electrode 49, the distance between the target tissue and the electrode 49 can be made longer. From this point, this embodiment is particularly effective if the coagulation range is to be changed in the target tissue. The other effects are the same as those of the above-mentioned second embodiment.

### (Eighth embodiment)

Fig. 14 is a partial sectional view showing the construction of the distal side of a treatment instrument of this embodiment. The construction of a treatment instrument 283 of this embodiment is different from the treatment instrument 223 of the second embodiment shown in Fig. 6 in the shape of the electrode. Thus, only the difference will be described, and the same reference numerals are given to the same components as those in the second embodiment and the description will be omitted.

In this embodiment, the electrode 20 is not formed in the cylindrical shape as shown in the above-mentioned second embodiment, but the electrode is formed in the L-shaped rod member, which is the difference.

Specifically, as shown in Fig. 14, an electrode 50 in this embodiment is formed of the L-shaped rod member having conductivity extending from a part of the outside of the nozzle portion 23 toward the distal side and bent at the position overlapping in a plane with the nozzle portion 23 far from the distal end of the nozzle portion 23. That is, a distal end 50s of the electrode 50 is located closer to the distal side than the distal face 23s of the nozzle portion 23.

According to this construction, since the discharge is carried out from the distal end 50s of the L-shaped electrode 50, the shape of the electrode can be simplified as compared with the above-mentioned second embodiment, which has an effect to reduce the manufacturing cost of the electrode. Also, since the injection range of the liquid W can be limited, the coagulation range for the target tissue can be narrowed. The other effects are the same as those of the above second embodiment.

### (Ninth embodiment)

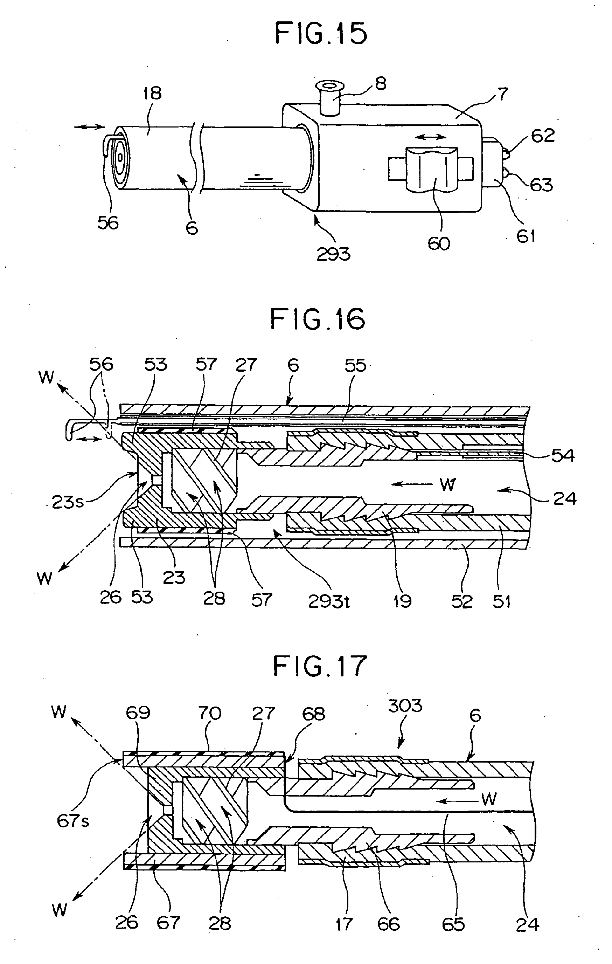

Fig. 15 is a perspective view showing the construction of a treatment instrument showing this embodiment, and Fig. 16 is a partial sectional view of the distal side of the treatment instrument in Fig. 15.

The construction of a treatment instrument 293 of this embodiment is different from the treatment instrument 283 of the eighth embodiment shown in Fig. 14 in the points that the L-shaped electrode is capable of moving forward/backward and two electrodes are provided at the treatment instrument 293. Thus, only the difference will be described, and the same reference numerals are given to the same components as those in the eighth embodiment and the description will be omitted.

As shown in Fig. 16, an outer tube 52 covers the outside of the nozzle portion 23, the tube connection portion 19 and an inner tube 51 capable of moving forward/backward so that a space is provided between it and a treatment instrument body 293t, which is a tubular member. The inner tube 51 has the same construction and connecting mode as the above-mentioned tube 17 (See Fig. 3).

At the distal face 23s of the nozzle portion 23, a first electrode 53 substantially in the ring shape is provided projecting toward the distal side. Also at the proximal side of the nozzle portion 23, the tube connection portion 19 is connected as mentioned above, and a lead wire 54, which is an electric conductive member, for the first electrode is electrically connected to the tube connection portion 19.

Moreover, in a space between the outside of the treatment body 293t and the outer tube 52, an electrode support rod 55, which is an L-shaped conductive rod member, is provided which is capable of moving forward/backward between the distal side and the proximal side of the treatment instrument 293 and covered by an insulating coating, and at the distal end of the electrode support rod 55, a second electrode 56 is provided.

The electrode support rod 55 is fixed by a positioning member, not shown, so as to move forward/backward, so that it is not displaced in the circumferential direction. Also, the second electrode 56 is formed at the position of the electrode support rod 55 which is bent at the position overlapping in a plane with the nozzle portion 23 away from the distal end of the nozzle portion 23.

Outside of the nozzle portion 23, an insulating layer 57 which electrically insulates the nozzle portion 23 from the electrode support rod 55 is provided. That is, the nozzle portion 23 is electrically insulated from the second electrode 56.

As the insulating layer 57, insulating coating such as ceramics, resin or the like or a tube shaped member made of the similar material stuck to the outside of the nozzle portion 23 may be used.

As shown in Fig. 15, an electrode operation lever 60 capable of sliding movement and a cable connection portion 61 are further provided at the proximal portion 7. To the electrode operation lever 60, a part of the electrode support rod 55 is connected.

At the cable connection portion 61, a plug 62 for a first electrode and a plug 63 for a second electrode are provided. To the plug 62 for the first electrode, the lead wire 54 for the first electrode is electrically connected. Also, to the plug 63 for the second electrode, the electrode support rod 55 is electrically connected.

Moreover, the high-frequency power source 4 is connected to each of the plugs 62, 63 by a conductive cable, not shown, and electricity is conducted either to the plug 62 for the first electrode or the plug 63 for the second electrode by the high-frequency power source 4.

Next, operation of the so constructed embodiment will be described.

First, when the electrode operation lever 60 is operated to be slid, the electrode support rod 55 and the second electrode 56 are moved forward/backward. That is, at the position where the second electrode 56 is moved to the proximal side shown by a two-dot chain line in Fig. 16, the second electrode 56 is moved to the position avoiding the liquid W injected from the nozzle hole 26.

On the other hand, at the position where the second electrode 56 is moved to the distal side shown by a solid line in Fig. 16, the second electrode 56 is moved to the position overlapping in a plane with the liquid W injected from the nozzle hole 26.

Here, when the first electrode 53 is energized, energization is performed in the state where the second electrode 56 is moved to the proximal side, that is, the second electrode 56 is moved to the position to avoid the liquid W. As a result, since discharge is performed from the ring-shaped first electrode 53, the coagulation range for the target tissue becomes relatively large.

Also, when energized from the second electrode 56, energization is performed in the state where the second electrode 56 is moved to the distal side, that is, the second electrode 56 is moved to the position overlapping in a plane with the liquid W.

In this case, since discharge is performed from the L-shaped second electrode 56, the coagulation range gets smaller. Also, in this case, it is possible to bring the second electrode 56 into contact with the living tissue so as to be used as a normal contact type electrode without atomizing the liquid W from the nozzle hole 26.

According to the above construction and operation, by selecting the electrode to perform discharge for the target tissue, the size of the coagulation range in the target tissue can be freely selected. The other effects are the same as those in the above-mentioned eighth embodiment.

### (Tenth embodiment)

Fig. 17 is a partial sectional view showing the construction of the distal portion of a treatment instrument showing this embodiment.

The construction of a treatment instrument 303 of this embodiment is different from the treatment instrument 223 of the second embodiment shown in Fig. 6 in the conducting method of the high-frequency current to the electrode. Thus, only the difference will be described, and the same reference numerals are given to the same components as those in the second embodiment and the description will be omitted.

As shown in Fig. 17, a lead wire 65, which is an electric conductive member, inserted through the flow passage 24 comes out from the outside surface of a tube connection portion 66 to the outside and is electrically connected to an electrode 67 at a connection portion 68 of the electrode 67. The construction of the tube connection portion 66 is the same as that of the above-mentioned tube connection portion 19, and the construction of the electrode 67 is the same as that of the above-mentioned electrode 20.

A nozzle portion 69 is provided on the inside surface of the electrode 67. Since the nozzle portion 69 is not energized, its material may be an electrically insulating material such as ceramics and resin. The other construction of the nozzle portion 69 is the same as those of the above-mentioned nozzle portion 23.

On the outside of the electrode 67, an insulating layer 70 may be provided with the purpose of concentrating discharge to a distal end 67s. The insulating layer 70 has the same construction as that of the insulating layer 57 shown in the above-mentioned ninth embodiment.

From above, the high-frequency current is transmitted in the order of the lead wire 65, the electrode 67 and the target tissue.

According to the above construction, since the nozzle portion 69 can be constructed from a material other than metal, the nozzle portion 69 can be manufactured inexpensively. The other effects are the same as those of the above-mentioned second embodiment.

### (Eleventh Embodiment)

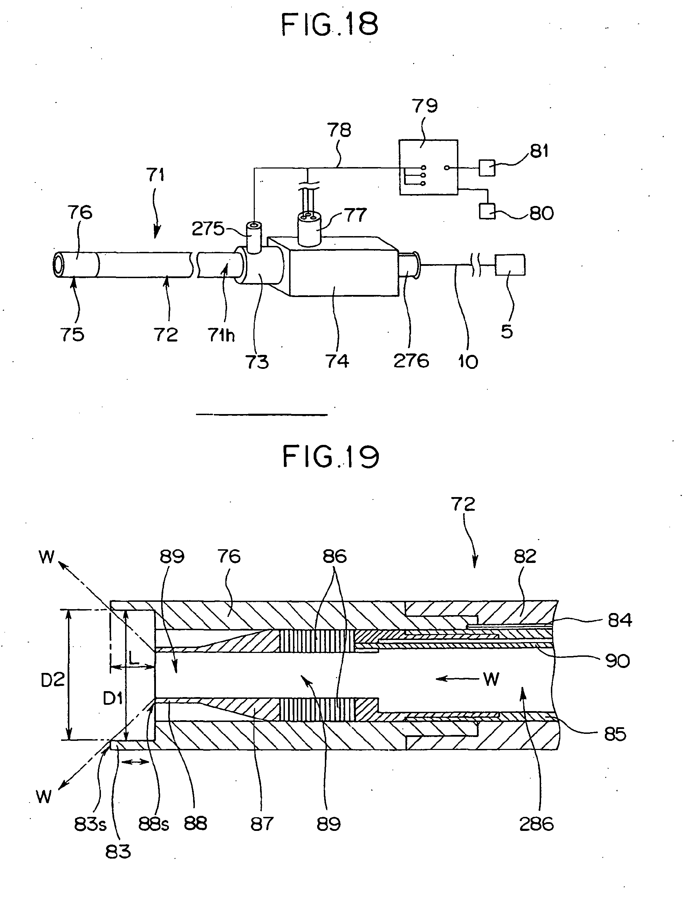

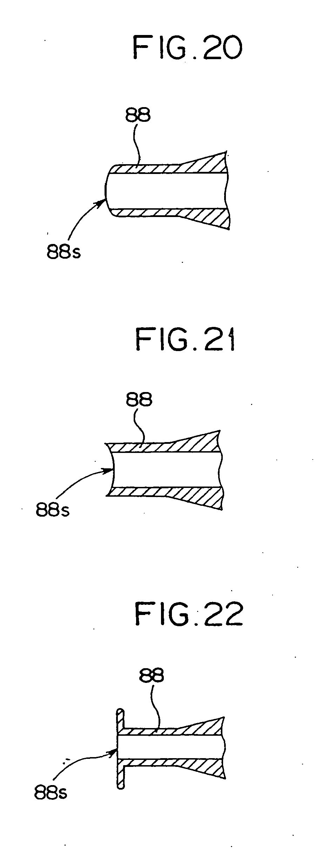

Fig. 18 is a block diagram showing a treatment instrument system provided with a treatment instrument showing this embodiment, Fig. 19 is a partial sectional view showing the construction of the distal side of the treatment instrument in Fig. 18, Fig. 20 is a partial sectional view showing a variation of the shape of a mist generation portion of the treatment instrument in Fig. 19, Fig. 21 is a partial sectional view showing another variation of the shape of a mist generation portion of the treatment instrument in Fig. 19, and Fig. 22 is a partial sectional view showing still another variation of the shape of a mist generation portion of the treatment instrument in Fig. 19.

The construction of the treatment instrument of this embodiment is different from the treatment instruments of the first embodiment shown in Figs. 1 to 5 in the point that the liquid W is injected from the distal end of the treatment instrument using an ultrasonic vibration. Thus, only the difference will be described, and the same reference numerals are given to the same components as those in the first embodiment and the description will be omitted.

As shown in Fig. 18, a treatment instrument 71 mainly comprises an elongated insertion portion 72 provided at a treatment instrument body 71 h, which is a tubular member, and capable of insertion/withdrawal with respect to a treatment channel 15 (See Fig. 1) of an endoscope 2, an operation portion 73 connected to the proximal side of the insertion portion 72, and a proximal portion 74 connected to the proximal side of the operation portion 73.

At the distal side of the insertion portion 72, a treatment portion 75 is constructed, and moreover, an electrode 76 is provided at the treatment portion 75.

At the operation portion 73, a high-frequency cable connection portion 275 is provided, while at the proximal portion 74, a connector 276 for liquid feed tube and an ultrasonic cable connection portion 77 are provided.

To the connector 276 for liquid feed tube, one end of a liquid feed tube 10 with the liquid feed pump 5 interposed at the middle position is connected.

To the high-frequency cable connection portion 275 and the ultrasonic cable connection portion 77, a high-frequency/ultrasonic driving power source (hereinafter simply referred to as a power source) 79 is connectable through a conductive cable 78.

To the power source 79, a foot switch 80 which controls output of a high-frequency current and an ultrasonic driving current, which is high-frequency electric energy, and a return electrode 81 used after output of the high-frequency current and the ultrasonic driving current are connected.

As shown in Fig. 19, the insertion portion 72 has a flexible tube 82 having an internal bore and at the distal end of the tube 82, a cylindrical elongated electrode 76 is connected. Also, a distal portion 83 of the electrode 76 is formed in a ring shape thinner than the electrode 76.

Also, in the internal bore of the tube 82, a lead wire 84, which is an electric conductive member, for conducting the high-frequency current is provided along the internal bore of the tube 82, and the distal end of the lead wire 84 is connected to the electrode 76, while the proximal end is connected to the high-frequency cable connection portion 275.

Also, along the internal bore of the tube 82, a flexible tube 85 is provided so as to move forward/backward and removable with respect to the tube 82.

Moreover, in the internal bore of the tube 85, a liquid feed passage 286 is constructed. At the distal side of the tube 85, a Langevin (electrostrictive) type cylindrical transducer 86 which generates ultrasonic vibration is connected, and at the distal side of the transducer 86, a cylindrical conical horn 87 which amplifies amplitude of the transducer 86 is connected and moreover, at the distal side of the horn 87, a tube-shaped mist generation portion 88 as a nozzle is connected.

The transducer 86 may be constructed by a magnetostrictive type transducer, other than a Langevin type transducer. Also, the shape of a distal end 88s of the mist generation portion 88 may be in the recessed or projecting R shape as shown in Fig. 20 or Fig. 21 or in the T shape as shown in Fig. 22 other than the end face shape shown in Fig. 19.

As the frequency of the transducer 86, M (mega) Hz level frequency is preferable to form atomization, but the frequency of 20 to 100 kHz is appropriate due to dimensional restriction and the like.

Also, a liquid feed hole 89 communicating with the liquid feed passage 286 is formed inside the transducer 86, the horn 87 and the mist generation portion 88. Thus, the mist generation portion 88 atomizes the liquid W which is supplied from the liquid feed hole 89 and to which ultrasonic vibration is applied by the transducer 86, the horn 87. The mist generation portion 88 carries out atomization so that the outer diameter of the liquid W at the distal end 83s of the distal portion 83 becomes D2.

Moreover, the distal end 88s of the mist generation portion 88 is located on the proximal side from the distal end 83s of the distal portion 83 of the electrode 76 by the distance L, and the inner diameter of the distal portion 83 is formed at D1. The inner diameter D1 is set equal to or larger than the outer diameter D2 of the liquid W (D1 ≥ D2).

Moreover, in the internal bore of the tube 85, a conductive cable 90 which supplies an ultrasonic driving current to the transducer 86 for driving the transducer 86 is provided along the liquid feed passage 286.

Next, operation of the so constructed embodiment will be described.

First, the liquid W is fed by the liquid feed pump 5 through the liquid feed tube 10, the connector 276 for liquid feed tube, the liquid feed passage 286 and the liquid feed hole 89 in this order, and at the same time as the liquid feeding, the transducer 86 is ultrasonically vibrated and the ultrasonic vibration is transmitted to the mist generation portion 88.

By this, at the distal end of the mist generation portion 88, the liquid W is atomized by vibration and after that, it is atomized from the mist generation portion 88 in front of the distal side. The distal end of the mist generation portion 88 constitutes a nozzle.

At this time, the liquid feed amount of the liquid W from the mist generation portion 88 and the amplitude of the transducer 86 are adjusted so that the outer diameter D2 of the liquid W at the distal end 83s of the distal portion 83 becomes equal to or smaller than the inner diameter D1 of the distal end 83s (D2 ≦ D1).

Also, at atomization, by conducting the high-frequency current from the power source 79 to the distal portion 83, discharge is performed toward the target tissue along the atomization. Also, since the tube 82 to which the electrode 76 is connected is capable of moving forward/backward with respect to the tube 85 by the operation portion 73, the position of the distal portion 83 with respect to the mist generation portion 88 can be freely adjusted.

According to the construction and operation of this embodiment, by using ultrasonic vibration for the atomization from the mist generation portion 88, atomization of the liquid W with smaller particle diameter is made possible.

If the particle diameter of atomization is reduced, the distance between the liquid particles in atomization is shortened and discharge is easily generated. Therefore, the distance between the distal portion 83 and the target tissue can be made larger. Also, since conductivity efficiency is improved, coagulation capability of the target tissue is improved.

Also, the distal end 88s of the mist generation portion 88 is separated from the distal end 83s of the distal portion 83 by the distance L. Discharge is generated from the distal portion 83, so that it is possible to reduce discharge from the mist generation portion 88, and ablation of the mist generation portion 88 can be prevented. Thus, the atomization shape of the liquid W is not changed over time but favorable coagulation can be obtained for the target tissue over a wide range all the time.

Also, since the position of the distal portion 83 can be adjusted by the operation portion 73, an optimal discharge state can be selected. Moreover, even if the distal portion 83 is worn, the distal portion 83 can be easily replaced and then, replacement of the distal portion 83 can be carried out economically.

Furthermore, by setting D2 ≦ D1, water drops will not be collected in the vicinity of the electrode 76, and a stable discharge state for the target tissue can be realized and as a result, favorable coagulation can be obtained for the target tissue over a wide range all the time.

Also, it is possible to cool the transducer 86 when the liquid W passes through the liquid feed hole 89.

Moreover, by forming the distal end 88s of the mist generation portion 88 in the shapes shown in Figs. 20 to 22, the atomization shape can be adjusted. That is, only by changing the shape of the distal end 88s of the mist generation portion 88, the size of the optimal coagulation range for the target tissue can be selected.

### (Twelfth embodiment)

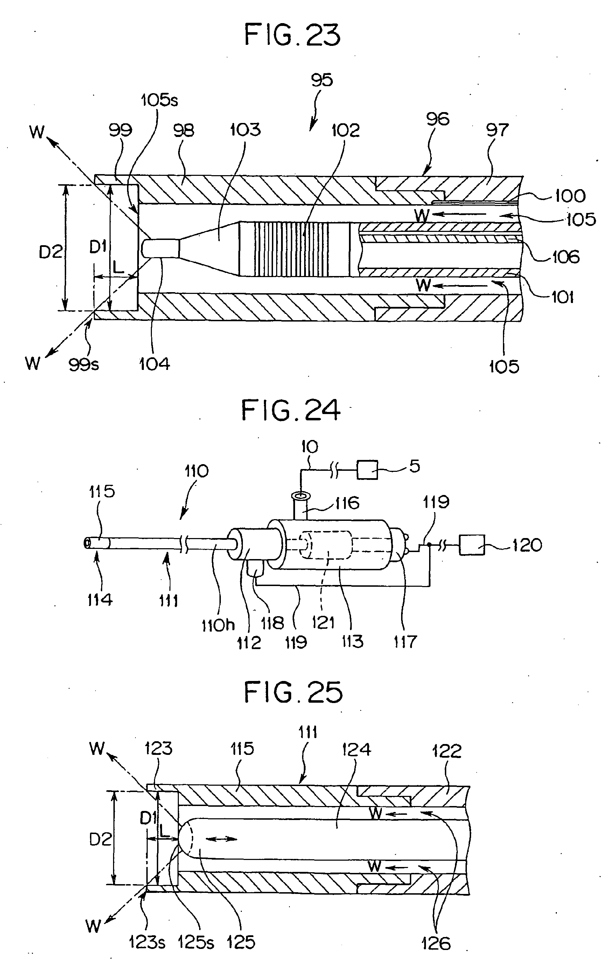

Fig. 23 is a partial sectional view showing the construction of the distal side of a treatment instrument showing this embodiment.

The construction of the treatment instrument of this embodiment is different from the treatment instrument of the eleventh embodiment shown in Figs. 18 to 22 in the point that the transducer is formed in the rod shape. Thus, only the difference will be described, and the same reference numerals are given to the same components as those in the eleventh embodiment and the description will be omitted.

As shown in Fig. 23, an insertion portion 96 of a treatment instrument 95 has a flexible tube 97 having an internal bore. At the distal end of the tube 97, a cylindrical and elongated electrode 98 is connected, and a distal portion 99 of the electrode 98 is formed in a ring shape thinner than the electrode 98.

Also, a lead wire 100 which is an electric conductive member for energizing a high-frequency to the electrode 98 is provided in the internal bore of the tube 97 along the tube 97, and the distal end of the lead wire 100 is connected to the electrode 98.

Moreover, in the internal bore of the tube 97, a flexible tube 101 is provided along the internal bore, and Langevin (electrostrictive) type transducer 102 which generates ultrasonic vibration is provided at the distal side of the tube 101.

Furthermore, at the distal side of the transducer 102, a conical horn 103 which amplifies amplitude is provided. At the distal side of the horn 103, a rod-shaped mist generation portion 104 is connected.

Also, the inner diameter D1 of a distal end 99s of the distal portion 99 is formed with the diameter equal to or larger than the outer diameter D2 (D1 ≧ D2) of the liquid W atomized from the mist generation portion 104 at the distal end 99s.

The transducer 102 may be constructed from a magnetostrictive transducer other than the Langevin type transducer. The shape of a distal end 104s of the mist generation portion 104 may be formed in the recess shape or T-shape. Also, a clearance to be a liquid feed passage 105 is provided between the tube 97 and the tube 101.

An injection port of the liquid feed passage 105 in the vicinity of the mist generation portion 104 constitutes a nozzle. The distal end 99s of the distal portion 99 is located protruding from a distal end 105s of the liquid feed passage 105 toward the distal side by the distance L.

In the internal bore of the tube 101, a conductive cable 106 is provided which supplies power to the transducer 102 in order to drive the transducer 102.

Next, operation of the so constructed embodiment will be described.

First, when the liquid W is fed through the liquid feed passage 105 to the vicinity of the mist generation portion 104 and at the same time, the transducer 102 is ultrasonically vibrated, atomization is generated by the mist generation portion 104. As a result, the liquid W is injected from the distal end 105s of the liquid feed passage 105.

At atomization of the liquid W, since the high-frequency current is conducted through the distal portion 99 from the high-frequency power source, discharge is performed toward the target tissue along the injection of the liquid W. The other operations are the same as those of the above-mentioned eleventh embodiment.

According to this construction and operation, since the transducer 102, the hom 103 and the mist generation portion 104 are solid, their manufacturing costs are lower as compared with the above-mentioned eleventh embodiment.

Also, if the distal end 104s of the mist generation portion 104 is made into the projecting or T-shaped shape, the atomization range can be widened, while if it is made into the recess shape, the atomization range can be narrowed. Therefore, only by changing the shape of the distal end 104s of the mist generation portion 104, the atomization shape can be adjusted, and the size of the coagulation range can be selected. The other effects are the same as those of the above-mentioned eleventh embodiment.

### (Thirteenth embodiment)

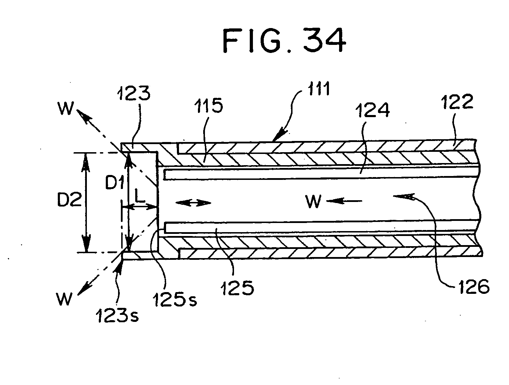

Fig. 24 is a block diagram showing a treatment instrument system provided with a treatment instrument of this embodiment, Fig. 25 is a partial sectional view showing the construction of the distal side of the treatment instrument of Fig. 24, and Fig. 34 is a partial sectional view showing a variation of a vibration probe in Fig. 25.

The construction of the treatment instrument of this embodiment is different from the treatment instrument of the twelfth embodiment shown in Fig. 23 in the point that the transducer is disposed on the proximal side of the treatment instrument. Thus, only the difference will be described, and the same reference numerals are given to the same components as those in the twelfth embodiment and the description will be omitted.

As shown in Fig. 24, a treatment instrument 110 is provided with a treatment instrument body 110h, which is a tubular member, and the treatment instrument body 110h mainly comprises an elongated insertion portion 111 capable of insertion/withdrawal with respect to the treatment channel 15 (See Fig. 1) of the endoscope 2, an operation portion 112 connected to the proximal side of the insertion portion 111, and a proximal portion 113 connected to the proximal side of the operation portion 112.

At the distal side of the insertion portion 111, a treatment portion 114 is comprised, and the treatment portion 114 is provided with an electrode 115.

At the proximal portion 113, a connector 116 for liquid feed tube and an ultrasonic cable connection portion 117 are provided. Also, at the operation portion 112, a high-frequency cable connection portion 118 is provided. Moreover, a transducer 121 is provided inside the proximal portion 113.

To the connector 116 for liquid feed tube, one end of a liquid feed tube 10 with the liquid feed pump 5 interposed at the middle position is connected. Also, to the ultrasonic cable connection portion 117 and the high-frequency cable connection portion 118, a high-frequency/ultrasonic driving power source 120 is connectable through a conductive cable 119.

As shown in Fig. 25, the insertion portion 111 has a flexible tube 122 having an internal bore, and to the distal end of the tube 122, a cylindrical electrode 115 is connected. Note that, as shown in Fig. 34, the electrode 115 may be provided in the inside of the tube 122.

At the distal end of the electrode 115, a distal portion 123 in the ring shape thinner than the electrode 115 is provided. The inner diameter D1 of a distal end 123s of the distal portion 123 is formed equal to or larger than the outer diameter D2 (D1 ≧ D2) of the liquid W at the distal end 123s atomized from the mist generation portion 125.

The proximal side of the tube 122 is connected to the operation portion 112. In the internal bore of the tube 122, an elongated and flexible vibration probe 124 connected to the transducer 121 is disposed along the internal bore. The tube 122 is capable of moving forward/backward by operating the operation portion 112 with respect to the vibration probe 124. The distal portion of the vibration probe 124 constitutes a mist generation portion 125, which is a nozzle.

Note that, as shown in Fig. 34, the vibration probe 124 may be formed in a cylindrical shape. In this case, the mist generation portion 125 is formed at the distal portion of the cylinder-shaped vibration probe 124.

Also, a distal end 125s of the mist generation portion 125 may be formed into the recess shape as shown by a broken line. Moreover, a clearance to be a liquid feed passage 126 is provided between the outside of the vibration probe 124 and the inside of the tube 122.

An injection port 126s in the vicinity of the distal end 125s of the mist generation portion 125 of the liquid feed passage 126 constitutes the nozzle. The injection port 126s is located on the proximal side away from the distal end 123s of the distal portion 123 by the distance L.

Next, operation of the so constructed treatment instrument of this embodiment will be described.

When the liquid W is fed through the liquid feed passage 126 to the vicinity of the mist generation portion 125 of the vibration probe 124 and at the same time, the transducer 121 is ultrasonically vibrated, atomization is generated at the mist generation portion 125.

According to this construction and operation, since the transducer 121 is provided at the proximal portion 113, the size of the transducer 121 can be increased and the range of choice is widened in frequency and amplitude of the vibration of the transducer 121, and the atomization particle diameter and shape suitable for discharge can be easily adjusted as compared with the twelfth embodiment, and as a result, favorable coagulation can be obtained.

Also, the atomization shape can be adjusted only by changing the distal shape of the mist generation portion 125, and the atomization range/coagulation range for the target tissue can be selected. The other effects are the same as those of the above-mentioned twelfth embodiment.

### (Fourteenth embodiment)

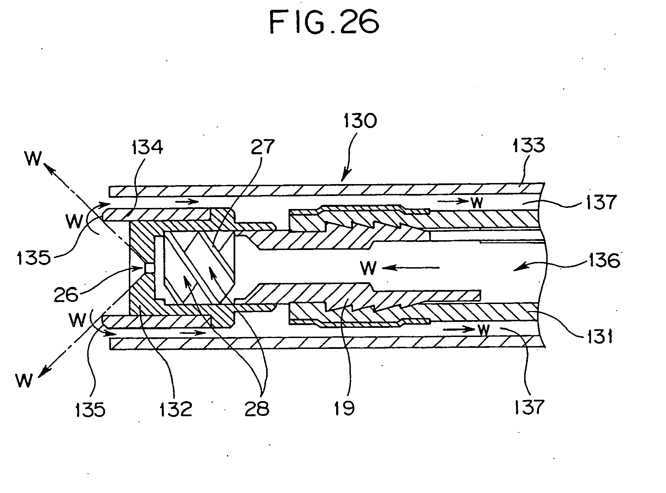

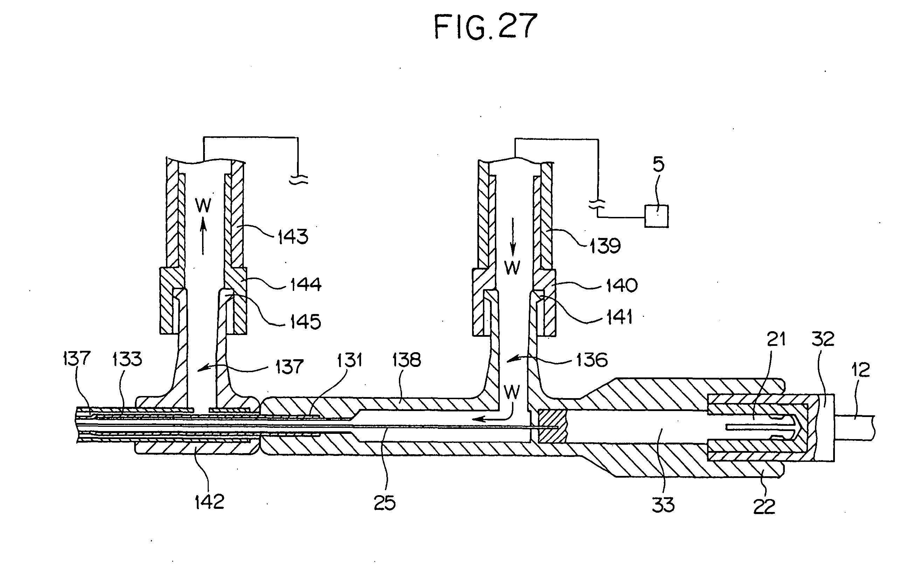

Fig. 26 is a partial sectional view showing the construction of the distal side of a treatment instrument showing this embodiment, and Fig. 27 is a partial sectional view showing the construction of the proximal side of the treatment instrument showing this embodiment.

The construction of the treatment instrument of this embodiment is different from the treatment instrument of the second embodiment shown in Fig. 6 in the point that a protective tube is provided on the outside of the treatment instrument and a passage for liquid suction is provided between the treatment instrument and the protective tube. Thus, only the difference will be described, and the same reference numerals are given to the same components as those in the second embodiment and the description will be omitted.

As shown in Fig. 26, a treatment instrument 130 has a flexible inner tube 131, and at the distal end of the inner tube 131, a nozzle portion 132 is provided through the tube connection portion 19. The inner tube 131 corresponds to the tube 17 of the second embodiment, and the nozzle portion 132 corresponds to the nozzle portion 23.

Also, a flexible outer tube 133, which is a protective tube covers the outside of the inner tube 131, capable of moving forward/backward and having a space between it and the inner tube 131, and the distal end of the outer tube 133 is located in the vicinity of a distal portion 135 of a cylindrical electrode 134 fixed to the outside of the nozzle portion 132. The electrode 134 corresponds to the electrode 20 of the second embodiment.

In the internal bore of the inner tube 131, a first passage 136 is formed, and a second passage 137, which is a suction passage, is formed in a space between the inner tube 131 and the outer tube 133.

As shown in Fig. 27, a proximal portion 138 is provided on the proximal side of the inner tube 131, and on the proximal portion 138, a connector 141 for liquid feed tube is provided to which a tube base 140 of a liquid feed tube 139 extended from the liquid feed pump 5 is connected. Inside of the connector 141 communicates with the first passage 136.

On the proximal side of the outer tube 133 and the distal side of the proximal portion 138, a suction body portion 142 is provided, and at the suction body portion 142, a connector 145 for suction tube is provided, to which a tube base 144 of a suction tube 143 is connected. The inside of the connector 145 communicates with the second passage 137. Also, the suction tube 143 is connected to a suction device, not shown.

Moreover, the suction body portion 142 is capable of adjustment of its relative position with respect to the electrode 134 of the outer tube 133 by being moved forward/backward.

Next, operation of the so constructed embodiment will be described.

First, the liquid W is fed by the liquid feed pump 5 from the first passage 136 to the nozzle portion 132. After that, the nozzle portion 132 atomizes the liquid W. Substantially at the same time, high-frequency current is conducted from the electrode 134 along the liquid W so as to perform discharge.

After that, even if water drops adhere to the vicinity of the electrode 134 through suctioning by the suction device, not shown, through the second passage 137, the adhering water drops are suctioned to the second passage 137. Also, the water drops adhering to the vicinity of the target tissue can be freely suctioned to the second passage 137.

According to this construction and operation, since the water drops in the vicinity of the electrode 134 can be removed, a stable discharge state can be realized for the target tissue and as a result, favorable coagulation over a wide range can be obtained for the target tissue all the time.

Also, since excess water drops around the target tissue can be suctioned and a liquid generating a cooling action can be removed, coagulability for the target tissue is further improved. The other effects are the same as those of the above-mentioned second embodiment.

### (Fifteenth embodiment)

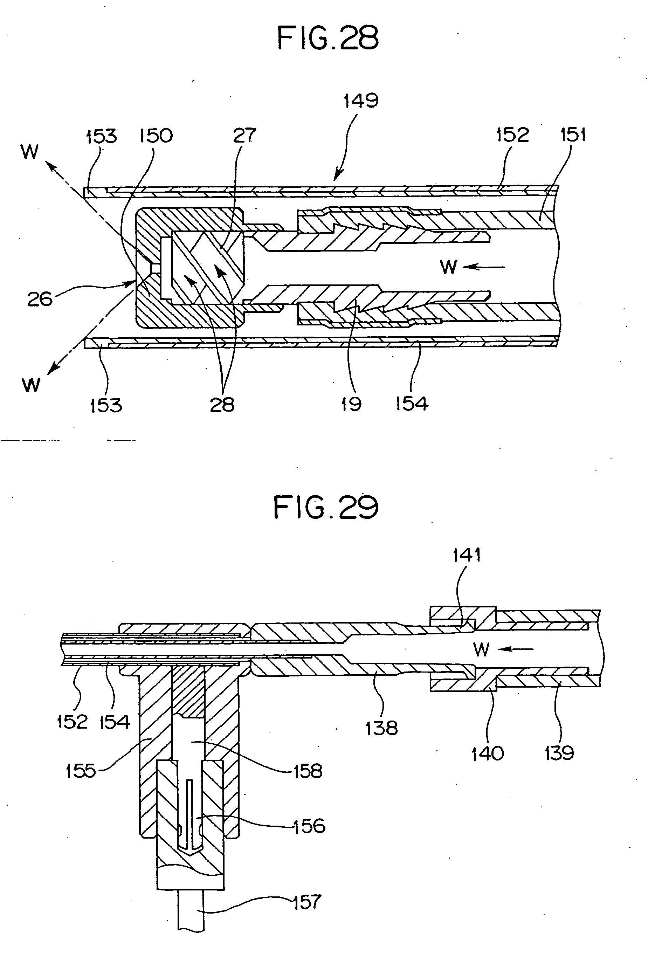

Fig. 28 is a partial sectional view showing the construction of the distal side of a treatment instrument showing this embodiment, and Fig. 29 is a partial sectional view showing the construction of the proximal side of the treatment instrument showing this embodiment.

The construction of the treatment instrument of this embodiment is different from the treatment instrument of the fourteenth embodiment shown in Figs. 26, 27 in the point that an electrode is provided at the distal end of a protective tube on the outside of the treatment instrument. Thus, only the difference will be described, and the same reference numerals are given to the same components as those in the fourteenth embodiment and the description will be omitted.

As shown in Fig. 28, a treatment instrument 149 has a flexible inner tube 151, and at the distal end of the inner tube 151, a nozzle portion 150 is provided through the tube connection portion 19. The inner tube 151 corresponds to the inner tube 131 of the fourteenth embodiment and the nozzle portion 150 corresponds to the nozzle portion 132.

At the distal end of an outer tube 152, which is a protective tube covering the outside of the inner tube 151 with a space, an electrode 153 is provided. On the inside surface of the outer tube 152, a conductive sheath 154 made of a flexible coil sheath or the like is provided, and the distal portion of the conductive sheath 154 is electrically connected to the electrode 153.

As shown in Fig. 29, the proximal portion of the outer tube 152 is connected to an outer tube operation portion 155.

A plug 156 is provided at the outer tube operation portion 155, and a conductive cable 157 to be connected to the high-frequency power source 4 (See Fig. 1) is connected to the plug 156. Also, to the plug 156, the proximal portion of the conductive sheath 154 is electrically connected through a plug body 158.

According to this construction, by operating the outer tube operation portion 155 forward/backward, the relative position of the electrode 153 with respect to the nozzle portion 150 can be adjusted. Thus, since an optimal discharge along the atomization can be selected only by adjusting the position of the electrode 153, favorable coagulation can be obtained over a wide range for the target tissue all the time. Also, since the electrode 153 can be replaced together with the outer tube 152, even if the electrode 153 is worn, replacement is easy and it is economical. The other effects are the same as those of the above-mentioned fourteenth embodiment.

### (Sixteenth embodiment)

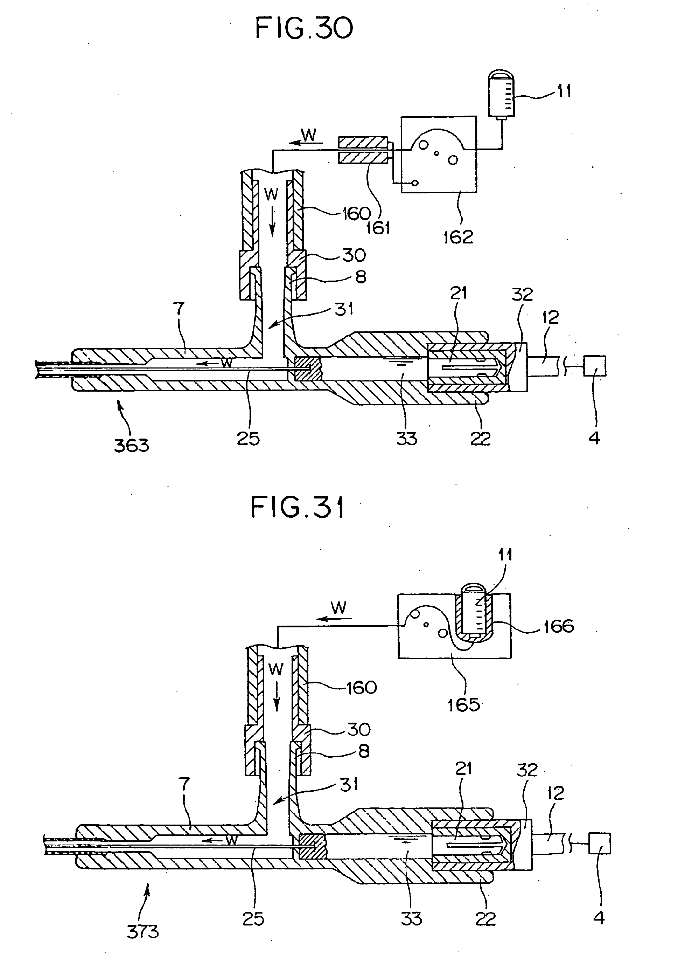

Fig. 30 is a partial sectional view showing a treatment instrument showing this embodiment together with the liquid feed pump.

The construction of a treatment instrument 363 of this embodiment is different from the treatment instrument 3 of the first embodiment shown in Figs. 1 to 5 in the point that the liquid to be injected is heated to a set temperature. Thus, only the difference will be described, and the same reference numerals are given to the same components as those in the first embodiment and the description will be omitted.

As shown in Fig. 30, at the middle position of a liquid feed tube 160, a tube heater 161, which is a heating device for heating the outside of the liquid feed tube 160 is provided. The liquid feed tube 160 corresponds to the liquid feed tube 10 of the first embodiment.

The tube heater 161 incorporates a heater portion; not shown, which generates heat by resistance heating, and the heater portion generates heat upon receipt of electric power from the liquid feed pump 162. As a result, the tube heater 161 heats the fed liquid W to a set temperature of 50 to 100°C.

According to this construction, since the liquid W is heated, a cooling effect by the liquid W on the target tissue can be reduced. Also, since the liquid W is at a high temperature and is easily evaporated, discharge from the electrode is generated easily and as a result, favorable coagulation can be obtained over a wide range. The other effects are the same as those of the above-mentioned first embodiment.

### (Seventeenth embodiment)

Fig. 31 is a partial sectional view showing a treatment instrument of this embodiment together with the liquid feed pump.

The construction of a treatment instrument 373 of this embodiment is different from the treatment instrument 363 of the sixteenth embodiment shown in Fig. 30 in the point that the tube heater is provided at the liquid feed pump. Thus, only the difference will be described, and the same reference numerals are given to the same components as those in the sixteenth embodiment and the description will be omitted.

In this embodiment, the liquid feed container 11 is provided at a liquid feed pump 165, and the liquid feed pump 165 incorporates a container heater 166, which is a heating device for heating the liquid feed container 11. The container heater 166 is to heat the liquid W to 50 to 100°C.

According to this construction, preparation can be simplified as compared with the sixteenth embodiment only by installing the liquid feed container 11 at the container heater 166.

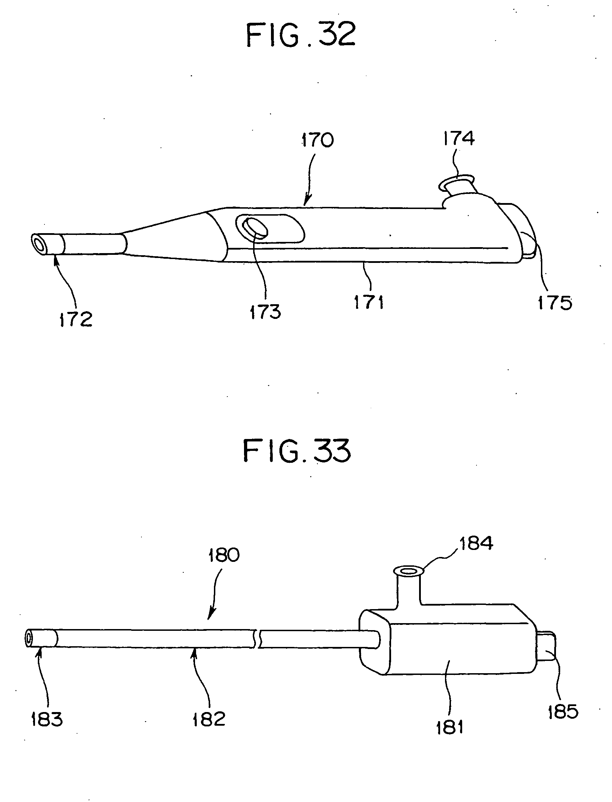

The treatment instrument described in the first to the seventeenth embodiments includes a handpiece for an abdominal surgery, for example. Fig. 32 is a perspective view showing the handpiece for an abdominal surgery.

As shown in Fig. 32, a handpiece 170 has a handpiece portion 171, and at the distal side of the handpiece portion 171, a treatment portion 172 is provided.

At the handpiece portion 171, a hand switch 173 which controls high-frequency energy, a liquid feed connector 174 and a conducting connector 175 are provided. The treatment portion 172 corresponds to the treatment portions 18, 75, 114 in the above-mentioned embodiments.

In this way, when the treatment instrument in the above-mentioned first to the seventeenth embodiments is applied to the handpiece, a user holds the handpiece portion 171 in hand and performs electric discharge while atomizing the liquid W from the treatment portion 172 for coagulation procedure for the target tissue.

According to this, when applied to the handpiece 170, since the handpiece portion 171 and the treatment portion 172 are close to each other, it has an advantage of suitability for a treatment of an abdominal surgery when the target tissue is close to the handpiece 170.

Alternatively, the treatment instruments described in the first to the seventeenth embodiments include a treatment instrument for a surgery under laparoscope, for example. Fig. 33 is a perspective view showing the treatment instrument for a surgery under laparoscope.

As shown in Fig. 33, a treatment instrument 180 has a handle portion 181, and at the distal side of the handle portion 181, an insertion portion 182 which can be inserted into a trocar and comprises a rigid shaft is provided.

At the distal end of the insertion portion 182, a treatment portion 183 is constructed. The treatment portion 183 corresponds to the treatment portions 18, 75, 114 of the above-mentioned embodiments.

When the treatment instrument of the above-mentioned first to the seventeenth embodiments is applied to a treatment instrument for surgery under laparoscope in this way, the user holds the handle portion 181, inserts the insertion portion 182 into the trocar, and performs electric discharge while atomizing the liquid from the treatment portion 183 under a laparoscope for coagulation procedure.

According to this, when applied to the treatment instrument 180 for surgery under laparoscope, since a rigid shaft is provided, it has an advantage that suitability for treatment under laparoscope can be obtained.

It is needless to say that the above-mentioned first to the seventeenth embodiments may be applied to other electrosurgical instruments using both the high-frequency electric energy and the conductive fluid for coagulating the surface layer of a living tissue by electric discharge.

## Claims

1. An electrosurgical instrument, comprising:
an elongated tubular member
a nozzle (23; 132; 150) provided at a distal side of the tubular member; said elongated tubular member having a flow passage therein for feeding a conductive fluid from a proximal side of said elongated tubular member to a proximal side of said nozzle; said nozzle being adapted to inject said conductive fluid from the distal end of the tubular member in an atomized state; and
an electrode (20; 49; 50; 53; 67; 98; 115; 134) provided to protrude distally from a distal face of the nozzle for discharging high-frequency electric energy supplied from a power source transmitted from a proximal side to the distal side of the tubular member through an electric conductive member (25) along the atomized-state conductive fluid injected from the nozzle (23; 132; 150).

2. The electrosurgical instrument according to Claim 1, further comprising a hole (26; 89) for injecting the atomized-state conductive fluid formed at a distal end of the nozzle (23; 132; 150).

3. The electrosurgical instrument according to Claim 1 or 2, wherein the electrode (20; 53; 67; 76; 98; 115; 134; 153) is formed of a cylindrical conductive member covering an outside of the nozzle (23; 132; 150).

4. The electrosurgical instrument according to Claims 2 or 3, wherein the electrode (20; 53; 67; 76; 98; 115; 134; 153) covers the outside of the nozzle (23; 132; 150) with the hole (26; 89) of the nozzle as a center axis (C) so that a center axis of the hole of the nozzle and a center axis of the electrode coincide with each other.

5. The electrosurgical instrument according to Claim 3 or 4, wherein the nozzle injects the conductive fluid so that an outer diameter of the conductive fluid at a distal end of the electrode as injected from the nozzle (23; 132; 150) is equal to or smaller than the inner diameter of the distal end of the electrode (20; 53; 67; 76; 98; 115; 134; 153).

6. The electrosurgical instrument according to Claim 3 or 4, wherein the inner diameter of the distal end of the electrode (20; 53; 67; 76; 98; 115; 134; 153) is formed equal to or larger than the outer diameter of the conductive fluid at the distal end of the electrode as injected from the nozzle (23; 132; 150).

7. The electrosurgical instrument according to any one of Claims 2 to 6,
wherein the hole (26; 89) of the nozzle (23; 132; 150) is formed so as to become wider from the proximal side to the distal side.

8. The electrosurgical instrument according to any one of Claims 2 to 7, further comprising a swirl member (27; 36) provided at a proximal side of the nozzle (23; 132; 150) in the inside of the tubular member, for introducing the conductive fluid into the hole (26; 89) of the nozzle by generating a swirling flow in the conductive fluid flowing to the inside of the tubular member.

9. The electrosurgical instrument according to Claim 8, wherein the swirl member (27; 36) is formed of a columnar member with a plurality of spiral flow passages (28) formed on an outside thereof.

10. The electrosurgical instrument according to Claim 8, wherein the swirl member (27; 36) is formed of a columnar member with a plurality of flow passages (28) and swirling portions formed on an outside and inside thereof.

11. The electrosurgical instrument according to any one of Claim 1, 2, 7 or 8, wherein the nozzle (23) is disposed in the inside of the tubular member in a state electrically insulated from the electrode (56).

12. The electrosurgical instrument according to any one of Claims 1 to 6, further comprising an ultrasonic transducer (86; 102) provided at the distal side of the tubular member (286), for applying ultrasonic vibration to the conductive fluid flowing into the inside of the tubular member.

13. The electrosurgical instrument according to any one of Claims 1 to 6, further comprising an ultrasonic transducer (121) provided at the proximal side of the tubular member, for applying ultrasonic vibration to the conductive fluid flowing into the inside of the tubular member (111).

14. The electrosurgical instrument according to any one of Claims 1 to 9, further comprising a protective tube covering (133) the tubular member (136) having a space (137) between the protective tube and the tubular member and capable of moving forward/backward to the distal side and the proximal side with respect to the tubular member,
wherein the space constitutes a suction passage connected to suctioning means, for suctioning the injected conductive fluid.

15. The electrosurgical instrument according to any one of Claims 1 to 14,
wherein the conductive fluid injected from the nozzle (23) is heated by a heating device (161) to a set temperature.

## Patentansprüche

1. Elektrochirurgisches Instrument, das aufweist:
ein röhrenförmiges Längselement,
eine an einer distalen Seite des röhrenförmigen Elements vorgesehene Düse (23; 132; 150), wobei das röhrenförmige Element einen Strömungskanal darin aufweist, um ein leitfähiges Fluid von einer proximalen Seite des röhrenförmigen Längselements einer proximalen Seite der Düse zuzuführen;
wobei die Düse dazu ausgebildet ist, das leitfähige Fluid vom distalen Ende des röhrenförmigen Elements in einem zerstäubten Zustand einzuspritzen; und
eine Elektrode (20; 49; 50; 53; 67; 98; 115; 134), die vorgesehen ist, um distal von einer distalen Fläche der Düse hervorzustehen, zum Abgeben hochfrequenter elektrischer Energie, die von einer Leistungsquelle zugeführt wird und die von einer proximalen Seite zur distalen Seite des röhrenförmigen Elements über ein leitfähiges elektrisches Element (25) entlang des aus der Düse (23; 132; 150) im zerstäubten Zustand eingespritzten leitfähigen Fluids übertragen wird.

2. Elektrochirurgisches Instrument nach Anspruch 1, das weiterhin eine Öffnung (26; 89) zum Einspritzen des leitfähigen Fluids im zerstäubten Zustand aufweist, die an einem distalen Ende der Düse (23; 132; 150) ausgebildet ist.

3. Elektrochirurgisches Instrument nach Anspruch 1 oder 2, wobei die Elektrode (20; 53; 67; 76; 98; 115; 134; 153) durch ein zylindrisches leitfähiges Element gebildet ist, das eine Außenseite der Düse (23; 132; 150) umgibt.

4. Elektrochirurgisches Instrument nach Anspruch 2 oder 3, wobei die Elektrode (20; 53; 67; 76; 98; 115; 134; 153) die Außenseite der Düse (23; 132; 150) mit der Öffnung (26; 89) der Düse als Mittenachse (C) umgibt, so dass eine Mittenachse der Düsenöffnung und eine Mittenachse der Elektrode zusammenfallen.

5. Elektrochirurgisches Instrument nach Anspruch 3 oder 4, wobei die Düse das leitfähige Fluid so einspritzt, dass ein Außendurchmesser des leitfähigen Fluids an einem distalen Ende der Elektrode in der aus der Düse (23; 132; 150) eingespritzten Form gleich oder kleiner ist als der Innendurchmesser des distalen Endes der Elektrode (20; 53; 67; 76; 98; 115; 134; 153).

6. Elektrochirurgisches Instrument nach Anspruch 3 oder 4, wobei der Innendurchmesser des distalen Endes der Elektrode (20; 53; 67; 76; 98; 115; 134; 153) gleich oder größer als der Außendurchmesser des leitfähigen Fluids am distalen Ende der Elektrode in der aus der Düse (23; 132; 150) eingespritzten Form ausgebildet ist.

7. Elektrochirurgisches Instrument nach einem der Ansprüche 2 bis 6, wobei die Öffnung (26; 89) der Düse (23; 132; 150) so ausgebildet ist, dass sie von der proximalen Seite zur distalen Seite breiter wird.

8. Elektrochirurgisches Instrument nach einem der Ansprüche 2 bis 7, das weiterhin ein Verwirbelungselement (27; 36) aufweist, das an einer proximalen Seite der Düse (23; 132; 150) im Inneren des röhrenförmigen Elements vorgesehen ist, um das leitfähige Fluid in die Öffnung (26; 89) der Düse einzuleiten, indem eine Verwirbelungsströmung in dem leitfähigen Fluid, das in das Innere des röhrenförmigen Elements strömt, erzeugt wird.

9. Elektrochirurgisches Instrument nach Anspruch 8, wobei das Verwirbelungselement (27; 36) durch ein säulenförmiges Element mit mehreren an einer Außenseite davon ausgebildeten spiralförmigen Strömungskanälen (28) gebildet ist.

10. Elektrochirurgisches Instrument nach Anspruch 8, wobei das Verwirbelungselement (27; 36) durch ein säulenförmiges Element mit mehreren an einer Außenseite und Innenseite davon ausgebildeten Strömungskanälen (28) und Verwirbelungsbereichen gebildet ist.

11. Elektrochirurgisches Instrument nach einem der Ansprüche 1, 2, 7 oder 8, wobei die Düse (23) im Inneren des röhrenförmigen Elements in einem von der Elektrode (56) elektrisch isolierten Zustand angeordnet ist.

12. Elektrochirurgisches Instrument nach einem der Ansprüche 1 bis 6, das weiterhin einen Ultraschallwandler (86; 102) aufweist, der an der distalen Seite des röhrenförmigen Elements (286) vorgesehen ist, um das in das Innere des röhrenförmigen Elements strömende leitfähige Fluid mit einer Ultraschallvibration zu beaufschlagen.

13. Elektrochirurgisches Instrument nach einem der Ansprüche 1 bis 6, das weiterhin einen Ultraschallwandler (121) aufweist, der an der proximalen Seite des röhrenförmigen Elements vorgesehen ist, um das in das Innere des röhrenförmigen Elements (111) strömende leitfähige Fluid mit eine Ultraschallvibration zu beaufschlagen.

14. Elektrochirurgisches Instrument nach einem der Ansprüche 1 bis 9, das weiterhin ein Schutzrohr aufweist, das das röhrenförmige Element (136) mit einem Freiraum (137) zwischen dem Schutzrohr und dem röhrenförmigen Element umgibt (133) und sich bezüglich des röhrenförmigen Elements zur distalen Seite und zur proximalen Seite vor und zurück zu bewegen vermag,
wobei der Freiraum einen Ansaugkanal bildet, der mit Ansaugmitteln zum Ansaugen des eingespritzten leitfähigen Fluids verbunden ist.

15. Elektrochirurgisches Instrument nach einem der Ansprüche 1 bis 14, wobei das aus der Düse (23) eingespritzte leitfähige Fluid durch eine Heizvorrichtung (161) auf eine festgelegte Temperatur erwärmt wird.

## Revendications

1. Instrument électrochirurgical, comprenant:
un élément tubulaire allongé
une buse (23; 132; 150) prévue au niveau d'un côté distal de l'élément tubulaire; ledit élément tubulaire allongé ayant un passage d'écoulement dans celui-ci destiné à délivrer un fluide conducteur d'un côté proximal dudit élément tubulaire allongé à un côté proximal de ladite buse;
ladite buse étant adaptée pour injecter un fluide conducteur depuis l'extrémité distale de l'élément tubulaire dans un état atomisé; et
une électrode (20; 49; 50; 53; 67; 98; 115; 134) prévue pour faire saillie de façon distale depuis une face distale de la buse pour évacuer une énergie électrique haute fréquence délivrée depuis une source d'énergie transmise d'un côté proximal au côté distal de l'élément tubulaire à travers un élément conducteur électrique (25) le long du fluide conducteur à l'état atomisé injecté depuis la buse (23; 132; 150).

2. Instrument électrochirurgical selon la revendication 1, comprenant en outre un trou (26 ; 89) destiné à injecter le fluide conducteur à l'état atomisé formé au niveau d'une extrémité distale de la buse (23 ; 132 ; 150).

3. Instrument électrochirurgical selon la revendication 1 ou 2, dans lequel l'électrode (20 ; 53 ; 67 ; 73 ; 98 ; 115 ; 134 ; 153) est formée d'un élément conducteur cylindrique recouvrant un extérieur de la buse (23 ; 132 ; 150).

4. Instrument électrochirurgical selon la revendication 2 ou 3, dans lequel l'électrode (20 ; 53 ; 67 ; 73 ; 98 ; 115 ; 134 ; 153) couvre l'extérieur de la buse (23 ; 132 ; 150) avec le trou (26 ; 89) de la buse comme axe central (C) de sorte qu'un axe central du trou de la buse et un axe central de l'électrode coïncident l'un avec l'autre.

5. Instrument électrochirurgical selon la revendication 3 ou 4, dans lequel la buse injecte le fluide conducteur de sorte qu'un diamètre externe du fluide conducteur au niveau d'une extrémité distale de l'électrode à mesure qu'il est injecté de la buse (23 ; 132 ; 150) est égal ou inférieur au diamètre interne de l'extrémité distale de l'électrode (20 ; 53 ; 67 ; 73 ; 98 ; 115 ; 134 ; 153).

6. Instrument électrochirurgical selon la revendication 3 ou 4, dans lequel le diamètre interne de l'extrémité distale de l'électrode (20 ; 53 ; 67 ; 73 ; 98 ; 115 ; 134 ; 153) est formé égal ou supérieur au diamètre externe du fluide conducteur au niveau de l'extrémité distale de l'électrode à mesure qu'il est injecté de la buse (23 ; 132 ; 150).

7. Instrument électrochirurgical selon l'une quelconque des revendications 2 à 6, dans lequel le trou (26 ; 89) de la buse (23 ; 132 ; 150) est formé de façon à devenir plus large en allant du côté proximal au côté distal.

8. Instrument électrochirurgical selon l'une quelconque des revendications 2 à 7, comprenant outre un élément de tourbillonnement (27 ; 36) prévu au niveau d'un côté proximal de la buse (23 ; 132 ; 150) à l'intérieur de l'élément tubulaire, destiné à introduire le fluide conducteur dans le trou (26 ; 89) de la buse en générant un écoulement tourbillonnant dans le fluide conducteur circulant vers l'intérieur de l'élément tubulaire.

9. Instrument électrochirurgical selon la revendication 8, dans lequel l'élément de tourbillonnement (27 ; 36) est formé d'un élément de type colonne avec une pluralité de passages d'écoulement en spirale (28) formés sur l'extérieur de celui-ci.

10. Instrument électrochirurgical selon la revendication 8, dans lequel l'élément de tourbillonnement (27 ; 36) est formé d'un élément de type colonne avec une pluralité de passages d'écoulement (28) et de parties tourbillonnantes formés sur un extérieur et un intérieur de celui-ci.

11. Instrument électrochirurgical selon l'une quelconque des revendications 1, 2, 7 ou 8, dans lequel la buse (23) est disposée à l'intérieur de l'élément tubulaire dans un état électriquement isolé de l'électrode (56).

12. Instrument électrochirurgical selon l'une quelconque des revendications 1 à 6, comprenant en outre un transducteur à ultrasons (86 ; 102) prévu au niveau du côté distal de l'élément tubulaire (286), destiné à appliquer des vibrations ultrasonores au fluide conducteur circulant à l'intérieur de l'élément tubulaire.

13. Instrument électrochirurgical selon l'une quelconque des revendications 1 à 6, comprenant en outre un transducteur à ultrasons (121) prévu au niveau du côté proximal de l'élément tubulaire, destiné à appliquer des vibrations ultrasonores au fluide conducteur circulant à l'intérieur de l'élément tubulaire (111).

14. Instrument électrochirurgical selon l'une quelconque des revendications 1 à 9, comprenant en outre un tube protecteur (133) couvrant l'élément tubulaire (136) comportant un espace (137) entre le tube protecteur et l'élément tubulaire et capable d'effectuer un mouvement de va-et-vient vers le côté distal et le côté proximal par rapport à l'élément tubulaire,
dans lequel l'espace constitue un passage d'aspiration raccordé au moyen d'aspiration, destiné à aspirer le fluide conducteur injecté.

15. Instrument électrochirurgical selon l'une quelconque des revendications 1 à 14, dans lequel le fluide conducteur injecté depuis la buse (23) est chauffé par un dispositif de chauffage (161) à une température établie.
